(19) Europäisches Patentamt / European Patent Office / Office européen des brevets

(11) **EP 4 058 413 B1**

(12) **EUROPEAN PATENT SPECIFICATION**

(45) Date of publication and mention
of the grant of the patent:
**16.04.2025   Bulletin 2025/16**

(51) International Patent Classification (IPC):
*C12M 3/06* (2006.01)      *C02F 3/08* (2023.01)
*C12M 1/00* (2006.01)      *C02F 3/10* (2023.01)
*C02F 3/34* (2023.01)      *C12M 1/12* (2006.01)

(21) Application number: **19842400.4**

(52) Cooperative Patent Classification (CPC):
**C02F 3/08; C02F 3/348; C12M 23/06; C12M 23/38;**
**C12M 27/14;** C02F 3/101; C02F 2303/20;
Y02W 10/10

(22) Date of filing: **04.12.2019**

(86) International application number:
**PCT/IB2019/060443**

(87) International publication number:
**WO 2021/094819 (20.05.2021 Gazette 2021/20)**

(54) **REACTOR FOR BIOFILM FORMATION AND RELATED PROCESS FOR BIOFILM FORMATION**

REAKTOR ZUR BIOFILMBILDUNG UND VERFAHREN ZUR BIOFILMBILDUNG

RÉACTEUR POUR LA FORMATION DE BIOFILMS ET PROCÉDÉ CONNEXE POUR LA
FORMATION DE BIOFILMS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.11.2019   PT 2019115912**

(43) Date of publication of application:
**21.09.2022   Bulletin 2022/38**

(73) Proprietor: **Universidade Do Porto**
**4099-002 Porto (PT)**

(72) Inventors:
• **BEZERRA GOMES, Inês**
**4099-002 Porto (PT)**
• **PEREIRA DE OLIVEIRA, Isabel Maria**
**4099-002 Porto (PT)**
• **DA FONSECA FERNANDES, Susana Maria**
**4099-002 Porto (PT)**
• **DIOGO CHAVES SIMÕES, Lúcia Conceição**
**4099-002 Porto (PT)**
• **VIEIRA SIMÕES, Manuel José**
**4099-002 Porto (PT)**

(74) Representative: **Patentree**
**Edificio Net**
**Rua de Salazares, 842**
**4149-002 Porto (PT)**

(56) References cited:
JP-A- 2007 282 629      US-A- 5 728 577

• GOMES I.B. ET AL: "The action of chemical and mechanical stresses on single and dual species biofilm removal of drinking water bacteria", SCIENCE OF THE TOTAL ENVIRONMENT, vol. 631-632, 1 August 2018 (2018-08-01), AMSTERDAM, NL, pages 987 - 993, XP093054690, ISSN: 0048-9697, DOI: 10.1016/j.scitotenv.2018.03.042
• GOMES I B ET AL: "An overview on the reactors to study drinking water biofilms", WATER RESEARCH, ELSEVIER, AMSTERDAM, NL, vol. 62, 3 June 2014 (2014-06-03), pages 63 - 87, XP029010204, ISSN: 0043-1354, DOI: 10.1016/J.WATRES.2014.05.039

- **JANG AM ET AL: "Measurement of chlorine dioxide penetration in dairy process pipe biofilms during disinfection", APPLIED MICROBIOLOGY AND BIOTECHNOLOGY, SPRINGER BERLIN HEIDELBERG, BERLIN/ HEIDELBERG, vol. 72, no. 2, 6 January 2006 (2006-01-06), pages 368 - 376, XP037015894, ISSN: 0175-7598, [retrieved on 20060106], DOI: 10.1007/S00253-005-0274-5**
- **PAUL STOODLEY ET AL: "Biofilms in medicine, industry and environmental biotechnology: Characteristics, analysis and control", 1 January 2013 (2013-01-01), XP055710751, Retrieved from the Internet <URL:https://www.researchgate.net/ publication/292460428_Biofilms_in_medicine_in dustry_and_environmental_biotechnology_Char acteristics_analysis_and_control/link/ 56c4891108ae736e7046eebb/download> [retrieved on 20200701]**

**Description**

**SCOPE OF THE INVENTION**

The present invention is related to a reactor for biofilm formation.

**STATE OF THE ART**

Biofilms are heterogeneous and complex structures of microorganisms, typically adhered on a surface and presenting sophisticated singular and collective behaviours.

The problems related to biofilms on industrial surfaces and processes are significant. For instance, the development of biofilms in drinking water distribution systems (DWDS) can cause pipe degradation, changes in the water organoleptic properties, but the main problem is related to the public health. Biofilms are the main responsible for the microbial presence in drinking water and can be reservoirs for pathogens.

In the food industry, biofilms are recognized as a source of recalcitrant contaminations, causing significant equipment damage, energy loss, food spoilage and are potential sources of public health problems due to outbreaks of foodborne pathogens.

Therefore, the understanding of the mechanisms underlying biofilm formation and its behaviour are of utmost importance in order to create effective control strategies.

Accordingly, there has been a great deal of research to better understand the mechanisms that promote biofilm development and to identify efficient control strategies. However, effective strategies for biofilm inactivation, removal from surfaces and regrowth prevention are not clearly identified. This is in part due to the absence of standardized assays for biofilm formation and control, particularly for industrial biofilms.

Various types of reactors are available to study biofilm formation and their control, as described in Gomes et al. "An overview on the reactors to study drinking water biofilms". Water Research 62 (2014) 63-87. Several devices, for example the biofilm annular reactor, the concentric cylinders reactor, the flow cell reactor, the Propella™ reactor, the rotating cylinder reactor, and the CDC biofilm reactor, were developed to study biofilms autonomously from a DWDS or other industrial facilities where biofilm formation may be critical. These devices allow the test of different conditions trying to mimic industrial environments or DWDS and they can be fed with tap water, enriched water or appropriate medium. In fact, these devices may be used as DWDS models to achieve a diversity of goals. However, they were used mostly in laboratorial experiments and have some disadvantages, which are detailed below.

> 1. **Biofilm annular reactor (BioSurface Technologies):** The biofilm annular reactor can operate as an open-/continuous system and has been used for several decades for biofilm formation under turbulent flow conditions. This reactor, also known as Rototorque, is constituted by two cylinders, one static external cylinder that can be of the actual pipe material and another rotating internal cylinder whose speed is controlled by a motor. The inner cylinder supports up to 20 slides/coupons used to sample the biofilm. The rotation of the inner cylinder is controlled in order to define the desired shear stress.
>
> 2. **Concentric cylinders reactor (CCR):** The CCR was firstly described and used to study biofilm formation in the dairy industry. The CCR is composed by four rotating cylinder pipes and four stationary cylinder chambers. The chambers can be fed with an appropriate medium and the volume inside the chambers is constant and controlled with the help of external pumps, being the feeding ports different from the outlet and sampling ports. The shear stress is controlled with the rotational velocity and radius of the cylinders. This reactor allows the simultaneous generation of different shear rates on the same inoculating population, but not with the same water phase as the four chambers are fed independently.
>
> 3. **Flow cell reactor:** This reactor, also known as the Robbins device, consists on a duct segment where removable coupons are inserted in the inner wall, whose allows biofilm sampling over time. This system may present different configurations: the modified Robbins device - a semicircular duct with some coupons (only the upper face contacts with water) located on the flat wall and the flow pass-through the duct from the bottom to the top; a parallel plate flow cell reactor, which consists in a rectangular flow channel with small removable coupons inside.
>
> 4. **Propella™ reactor:** The Propella™ reactor has two concentric cylinders in which a propeller pushes the liquid down through the inner tube and then up through the annular section between both cylinders. It is a perfectly mixed reactor and the fluid velocity, the hydraulic residence time and the flow rate are controlled by the rotation speed of the propeller. Coupons are usually located on the outer tube facilitating the sampling process and, in some cases, the removal of coupons does not change the flowing conditions.
>
> 5. **Rotating cylinder reactor (RCR):** The RCR is a 5 L reactor with three cylinders immersed in a bacterial suspension. An overhead stirrer provides the simultaneous rotation of the three cylinders connected by a synchronizing belt. The cylinders can be made of different materials and provide a large sampling area.

6. **CDC biofilm reactor® (BioSurface Technologies):** The CDC biofilm reactor has eight coupon holders supported by a ported lid with each holder containing three coupons. The lid with the holders is mounted in a 1 L reactor and the agitation is ensured by placing the reactor on a controlled stirrer plate, providing a constant rotation of a baffle.

The biofilm annular reactor, the CCR reactor, the flow cell reactor, the Propella™ reactor, the RCR reactor and the CDC biofilm reactor have some common limiting aspects and disadvantages, related to the geometrical configuration of said reactors that can change the flow patterns, leading to a non-ideal mixing and a non-uniform biofilm formation. Moreover, it is not feasible to apply different chemical treatments simultaneously in these reactors, under controlled hydrodynamic conditions. It should be also noted that the biofilm annular reactor, the flow cell reactor, the Propella™ reactor and the CDC biofilm reactor use flat coupons lacking sufficient sampling surface area. As specific disadvantages of the CCR reactor, it is possible to mention the limited number of materials that can be tested - because only one surface material can be tested per experiment; the difficult sampling process; and the impossibility of performing replicates in the same experiment. On the other hand, in the Propella™ reactor, the biofilm development is difficult to follow, due to the non-transparent materials of the reactor. In the RCR reactor, the main disadvantages are the low number of sampling cylinders, only up to three, and a high working volume (5 L). Additionally, mechanical treatment is difficult to apply in the CDC biofilm reactor, because it is difficult to determine accurately the shear stress applied.

Other laboratory devices, such as the microtiter plates, Calgary biofilm reactor, the drip flow biofilm reactor and the rotating disk reactor were developed to allow the study of biofilm formation and control under specific conditions, in order to fill the gap on the limitations of previously described reactors. However, these reactors were developed to study medical biofilms as analyzed by Gomes et al. "Standardized reactors for the study of medical biofilms: a review of the principles and latest modifications". Critical Reviews in Biotechnology 38 (2018) 657-670. Their limitations to test industrial biofilms are significant as detailed below.

7. **Microtiter Plates:** The microtiter plates reactor is nowadays the most frequently used reactor system for studying biofilm formation. This reactor can be used as a rapid and simple method to screen simultaneously the effect of high numbers of different parameters on biofilm formation.

8. **Calgary biofilm reactor:** This reactor is a simple device originally designed as a test for the assessment of antimicrobials efficacy and can also be used as a rapid and simple method to screen simultaneously different parameters.

9. **Drip flow biofilm reactor:** This reactor consists of four completely separated yet parallel channels, each one with an individual lid fixed with screws. The individual lids are important to keep the aseptic conditions during the sampling process. Each channel contains a coupon that may be made of a variety of materials.

10. **Rotating disk reactor:** The rotating disk reactor consists of a 1 L reactor with an effluent port located at approximately the 250-mL mark. The lid of the reactor has four inlet ports, one for inoculum and three for liquid medium or gas. There is a magnetically driven rotor, made from PTFE and rubber, which is placed at the bottom of the vessel. The rotor holds six removable coupons that are the substrate for biofilm formation.

Specifically, the disadvantages of microtiter plates and Calgary biofilm reactor are related to the operating conditions that do not allow a proper similarity with real conditions of biofilm formation or biofilm inactivation in industrial systems. Moreover, it is not possible to properly control the hydrodynamic conditions. Other limitations are related to its relatively low working volume and sampling area for biofilm analysis. Besides, its operating conditions are limited to a batch mode. The drip flow biofilm reactor and the rotating disk reactor have some common limiting aspects and disadvantages, due to the use of flat surfaces and the flow changes in the boundaries of the adhesion surfaces. Moreover, these reactors do not have a significant sampling area and it is not feasible to evaluate different hydrodynamic conditions or even to use mechanical treatments for biofilm removal. The operating conditions of these reactors do not allow a proper similarity with real conditions of biofilm formation or biofilm inactivation.

A further state of the art analysis also reveals some devices were developed to study and monitor biofilms in pilot and real DWDS, such as the Pennine Water Group coupon and the Bioprobe monitor. **Pennine Water Group (PWG) coupon:** The PWG coupon can be inserted directly into pipes, and is comprised of two parts, an "outer coupon" and an "insert". The outer coupon retains the curvature of the pipe and fits into a hole made in a removable and flanged identical pipe section. The coupon is fixed with a gasket to a section pipe. The insert is engineered flat to allow microscopic analysis and it fits inside the outer coupon in a way to allow the outer surface to be in direct contact with the water.

**Bioprobe monitor:** The Bioprobe monitor consists on a pipe where a coupon holder (denominated acetal) is inserted, being the coupon surface flushed with the pipe wall. The bioprobe monitor was specifically designed to study biofilm growth within a pipe system.

The PWG coupon and the Bioprobe monitor have some common limiting aspects and disadvantages, such as the reduced sampling area provided by the employed coupons, the limitation and difficulty in controlling the operational conditions and the change of the flow pattern due to the geometrical configuration of the said reactors. Besides that, it is not possible to apply simultaneously different chemical treatments for biofilm inactivation and removal in these reactors.

Gomes et al. "An overview on the reactors to study drinking water biofilms", Water Research 62 (2014); 63-87 discloses reactors to study drinking water biofilms, in particular devices that allow biofilm formation under controlled conditions of physical (flow velocity, shear stress, temperature, type of pipe material, etc), chemical (type and amount of nutrients, type of disinfectant and residuals, organic and inorganic particles, ions, etc) and biological (composition of microbial community - type of microorganism and characteristics) parameters, ensuring that the operational conditions are similar as possible to in drinking water distribution systems conditions in order to achieve results that can be applied to the real scenarios.

Jang et al. "Measurement of chlorine dioxide penetration in dairy process pipe biofilms during disinfection", Applied Microbiology and Biotechnology 72 (2006); 368-376 relates to a rotating drum biofilm annular reactor, consisting of a concentric outer cylinder (inner diameter of 11.7 cm) and a rotating inner cylinder with a total height of 25.4 cm. The reactor contained 40 removable flat stainless steel coupons on its inner wall to permit sampling of the biofilms.

Stoodley and Warwood "Use of flow cells and annular reactors to study biofilms" In: Biofilms in medicine, industry and environmental biotechnology: Characteristics, analysis and control (Len P, Moran AP, Mahony T, Stoodley P, O'Flaherty V (eds)), 2003, IWA Publishing, provides an analysis of various types of biofilm reactors, concentrating on the use of flow cells and the annular reactor for biofilm research and monitoring.

Gomes et al. "The action of chemical and mechanical stresses on single and dual species biofilm removal of drinking water bacteria". Science of the Total Environment 631-632 (2018) 987-993 discloses a rotating cylinder reactor used in drinking water biofilm studies with polyvinyl chloride (PVC) as substratum.

The reactor described and claimed in the present invention, was developed to overcome the limitations of the available biofilm reactors, allowing reliable studies of biofilm formation on cylindrical surfaces and their inactivation and removal, which are an integrated part of the said reactors. The said reactors may be operated under controlled conditions mimicking the ones found on industrial surfaces, particularly the conditions influenced by physical variables (shear stress, temperature, type of pipe material), chemical variables (type and amount of nutrients, type and concentration of disinfectant and residuals, organic and inorganic particles and ions) and biological variables (composition and density of the microbial community).

The advantages related to the reactors according to the present invention are significant such as their ideal and well mixed conditions, with the absence of Taylor vortices; their higher surface area for biofilm formation; the possibility of producing larger biofilm amounts for further analysis; the possibility of using simultaneously different materials for biofilm formation; the easy sampling steps; the reduction of changes in the flow patterns on the surface where biofilms are formed; and the possibility of applying different chemical and mechanical treatments per assay. Those are aspects that will be evident for a person skilled on the art in the detailed description and in the embodiments represented in drawings.

## BRIEF DESCRIPTION OF THE DRAWINGS

In order to promote an understanding of the principles according to the embodiments of the present invention, reference will be made to the embodiments shown in the figures and to the language used to describe them. In any event, it is to be understood that there is no intention to limit the scope of the present invention to the content of the figures, the invention being defined in the appended claims. Any subsequent changes or modifications of the inventive features herein and any further applications of the principles and embodiments of the invention illustrated which would normally occur to a person skilled in the art having the possession of this disclosure are within the scope of the claimed invention as defined in the appended claims.

Fig. 1 - a perspective view of the head (1);
Fig. 2 - a perspective view of the reactor for biofilm formation (3);
Fig. 3 - a perspective view of the reactor for biofilm inactivation and removal from the adhesion surface (5) (outside the subject-matter of the claims but useful for understanding the disclosure);
Fig. 4 - a perspective view of the feed and drainage cover (11);
Fig. 5 - a perspective view of the cover (16);
Fig. 6 - a perspective view of the interconnected base (15);
Fig. 7 - a perspective view of the base (17);
Fig. 8 - a sectional view of the reactor for biofilm formation (3); and
Fig. 9 - a sectional view of the reactor for biofilm inactivation and removal from the adhesion surface (5) (outside the subject-matter of the claims but useful for understanding the disclosure).

List of reference numbers:

- a head (1);

- a platform for biofilm formation (2);
- a reactor for biofilm formation (3);
- a platform for biofilm inactivation and removal from the adhesion surface (4);
- a reactor for biofilm inactivation and removal from the adhesion surface (5);
- a principal cover (6);
- a vertical hole (7);
- a vertical shaft (8);
- a cylinder (9);
- a system for rotating (10);
- a feed and drainage cover (11);
- a vertical vessel (12) and (12');
- a vertical and central feed vessel (13);
- a vertical support shaft (14) and (14');
- an interconnected base (15);
- a cover (16);
- a base (17);
- a vertical insertion hole (18) and (18');
- a vertical feed central hole (19);
- a vertical cavity (20) and (20');
- a vertical hole for support (21) and (21');
- a horizontal drainage hole (22);
- a sump feed (23);
- a bacterial culture (24);
- an initial nutrient medium (25);
- a diluted nutrient medium (26);
- a longitudinal hole (27);
- a base cavity (28);
- a base cavity for support (29);
- a horizontal leak hole (30);
- a base central cavity (31);
- a pulley (32);
- a pulley stretcher (33);
- a chemical agent (34); and
- a neutralizer solution (35).

## DETAILED DESCRIPTION OF THE INVENTION

The invention is defined by a reactor for biofilm formation comprising a head as defined in the claims.

Reference will now be made in detail to the preferred embodiments of the invention, examples of which are illustrated in the accompanying drawings. Whenever possible, the same reference numerals will be used throughout the drawings to refer to the same or like parts.

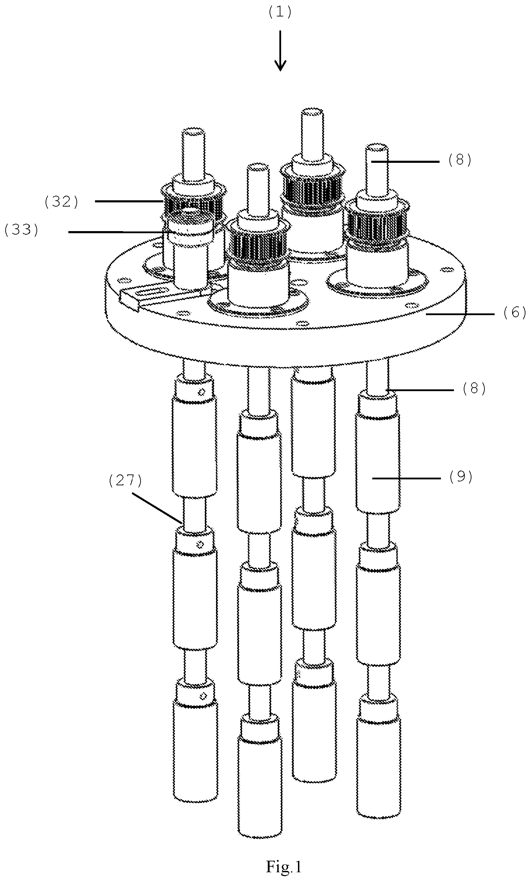

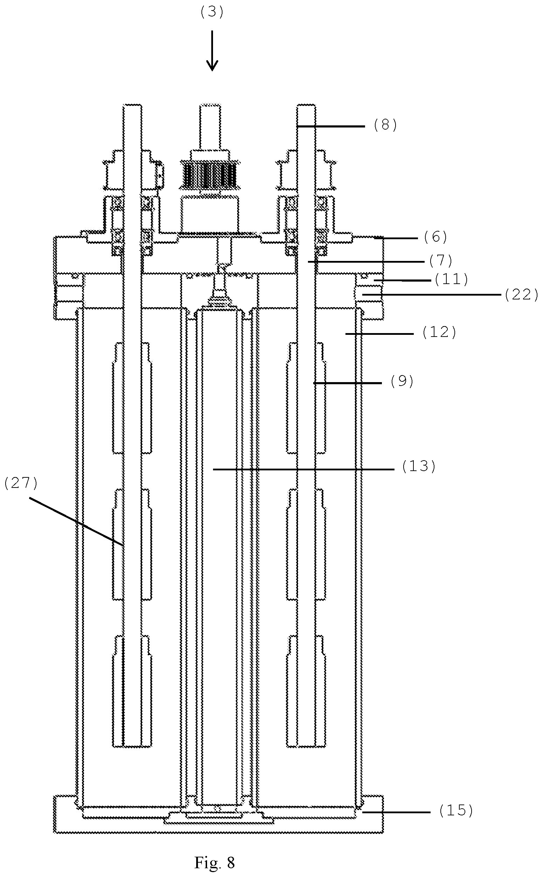

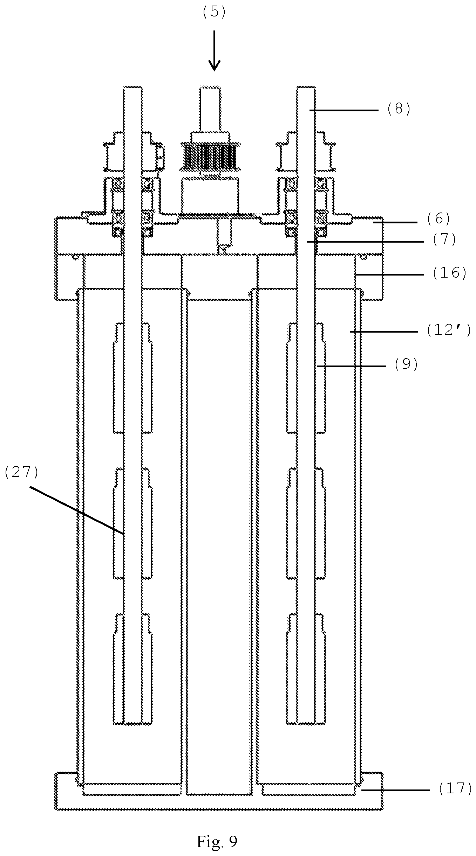

The present invention as defined in the claims refers to a head (1), represented in the figure 1, for a reactor for biofilm formation (3), comprises a principal cover (6) that includes at least a vertical hole (7), represented in the figures 8 and 9, wherein it is inserted a vertical shaft (8) into each vertical hole (7) and each vertical shaft (8) comprises at least a fixed cylinder (9), preferably three cylinders (9), wherein the cylinders (9) are arranged in series. Furthermore, the head (1) comprises a system for rotating (10) each respective vertical shaft (8) around its own axis.

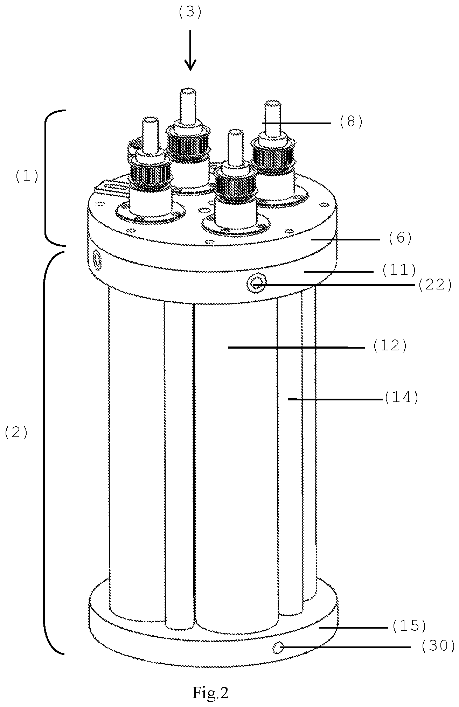

The present invention as defined in the claims refers to a reactor for biofilm formation (3), as shown in figures 2 and 8, wherein the head (1) is inserted into a platform for biofilm formation (2), wherein the platform for biofilm formation (2) comprises:

- a feed and drainage cover (11);
- a plurality vertical vessels (12), preferably four vertical vessels (12);
- a vertical and central feed vessel (13);
- a plurality of vertical support shafts (14), preferably four vertical support shafts (14); and
- an interconnected base (15).

Furthermore, each vertical shaft (8) of the invention, represented in the figures 1 and 8, is held in the principal cover (6) and is suspended inside each vessel (12).

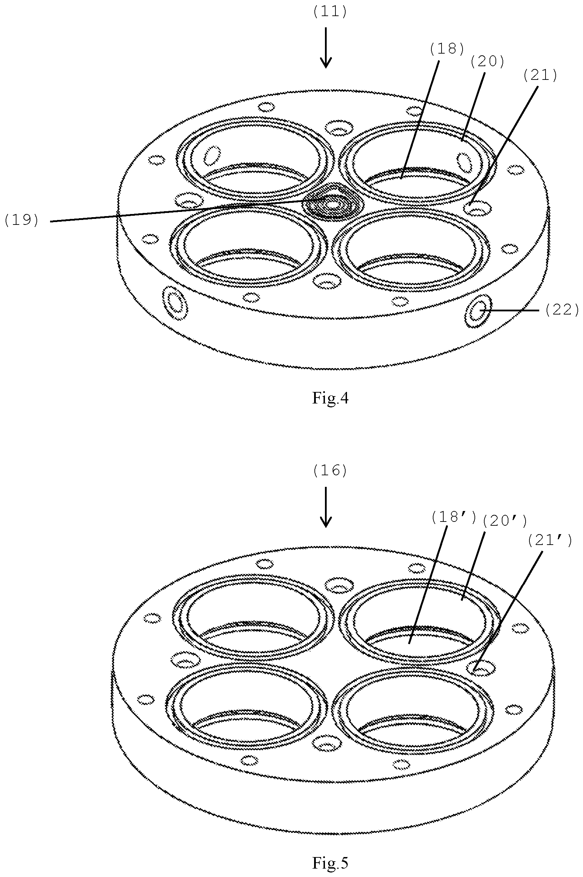

The feed and drainage cover (11), represented in the figures 2 and 4, comprises four horizontal drainage holes (22).

The reactor for biofilm formation (3), represented in the figures 2 and 8, operates under one of the selected conditions e.g. under batch, fed batch or continuous operating conditions.

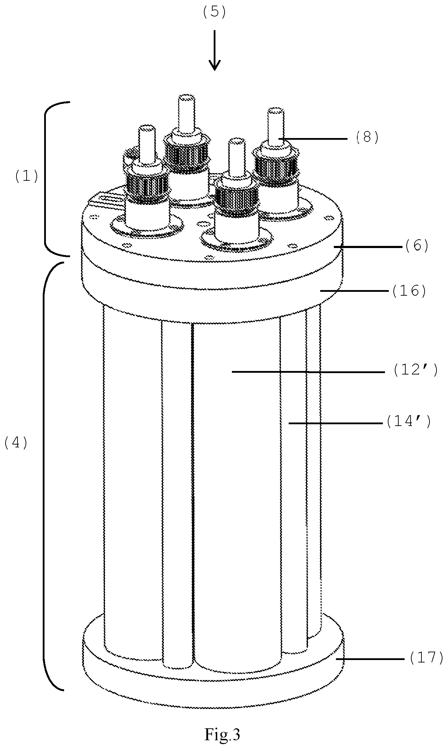

An embodiment outside the subject-matter of the claims but useful for understanding the disclosure, refers to a reactor for biofilm inactivation and removal from the adhesion surface (5), represented in the figures 3 and 9, comprising the head (1), wherein the head (1) is inserted into a platform for biofilm inactivation and removal from the adhesion surface (4), wherein the platform for biofilm inactivation and removal from the adhesion surface (4) comprises:

- a cover (16);
- at least a vertical vessel (12'), preferably four vertical vessels (12');
- at least a vertical support shaft (14'), preferably four vertical support shafts (14'); and
- a base (17).

Furthermore, each vertical shaft (8), represented in the figures 1 and 9, is held in the principal cover (6) and suspended inside each vessel (12').

In the present invention the feed and drainage cover (11), represented in the figure 4, comprises:

- at least a vertical insertion hole (18), for insertion of each respective vertical vessel (12), preferably four vertical insertion holes (18), for insertion of the respective four vertical vessels (12);
- a vertical feed central hole (19), for insertion of the respective vertical and central feed vessel (13);
- at least a vertical cavity (20), located at the bottom of the feed and drainage cover (11), for fitting the respective vertical vessel (12), preferably four vertical cavities (20), for fitting the respective four vertical vessels (12);
- at least a vertical hole for support (21), for insertion of each respective vertical support shaft (14), preferably four vertical holes for support (21), for insertion of the respective four vertical support shafts (14); and
- at least a horizontal drainage hole (22), located at side face of the feed and drainage cover (11), for drainage of the respective vertical vessel (12), preferably four horizontal drainage holes (22).

Furthermore, in the present invention, the feed and drainage cover (11) having:

- vertical insertion holes (18);
- vertical cavities (20); and
- horizontal drainage holes (22);

in equal number to the number of the vertical vessels (12).

Furthermore, in the present invention, the feed and drainage cover (11) further comprises a system for feeding and draining, not represented in the figures that includes:

- an external pump, not represented in the figures, for feeding through an external tube, not represented in the figures, the vertical and central feed vessel (13).

In a preferred embodiment of the present invention, the cover (16), represented in the figure 5, comprises:

- at least a vertical insertion hole (18'), for insertion of each respective vertical vessel (12'), preferably four vertical insertion holes (18'), for insertion of the respective four vertical vessels (12');
- at least a vertical cavity (20'), located at the bottom of the cover (16), for fitting the respective vertical vessel (12'), preferably four vertical cavities (20'), for fitting the respective four vertical vessels (12'); and
- at least a vertical hole for support (21'), for insertion of each respective vertical support shaft (14'), preferably four vertical holes for support (21'), for insertion of the respective four vertical support shafts (14').

Furthermore, in another preferred embodiment according to the present invention, the cover (16) has vertical insertion holes (18') and vertical cavities (20') in equal number to the number of the vertical vessels (12'), as represented in the figures 3 and 9.

In a preferred embodiment of the present invention, each vertical vessel (12') is tight and comprises:

- a tubular shape; and
- a working volume including:
- a diameter of about 27 to about 108 mm; and

- a length of about 136 to 544 mm.

In a preferred embodiment of the present invention the vertical vessel (12), represented in the figures 2 and 8, being tight and comprises:

- a tubular shape; and
- a working volume including:
- a diameter of about 27 to about 108 mm; and
- a length of about 136 to about 544 mm.

In according to one of the respective embodiments of the present invention, the vertical vessel (12) and (12') may present other different working volumes than the working volume referred to in the previous paragraph.

In according to one of the respective embodiments of the present invention, the vertical vessel (12) and (12') is manufactured with a translucent material, such as polymethyl methacrylate or glass.

In one of several embodiments of the present invention, the translucent material of the vertical vessel (12) and (12') allows visualization of biofilm formation and the biofilm removal from the adhesion surface.

Furthermore, in the present invention, each vertical vessel (12) is interconnected through the interconnected base (15) that comprises a sump feed (23) and each vertical vessel (12) is independent from one another and each vertical vessel (12) is fed by the vertical and central feed vessel (13), which is interconnected with all the vertical vessels (12) through the sump feed (23) of the interconnected base (15).

In a preferred embodiment of the present invention, the cylinders (9) comprise:

- a cylindrical shape;
- a sampling surface;
- a longitudinal hole (27) for insertion into the vertical shaft (8); and
- a fixation section.

And the cylinders (9) being manufactured using a material selected from the list consisting of:

- metals;
- metallic alloys;
- plastics;
- glass; and
- coatings and surface coats, wherein the coatings and surface coats comprising a nanomaterial or a chemically modified surface.

Furthermore, the cylinders (9) are fixed in the vertical shaft (8), preferably being at a distance of about 10 mm from each other; and the cylinders (9) rotate around its own axis.

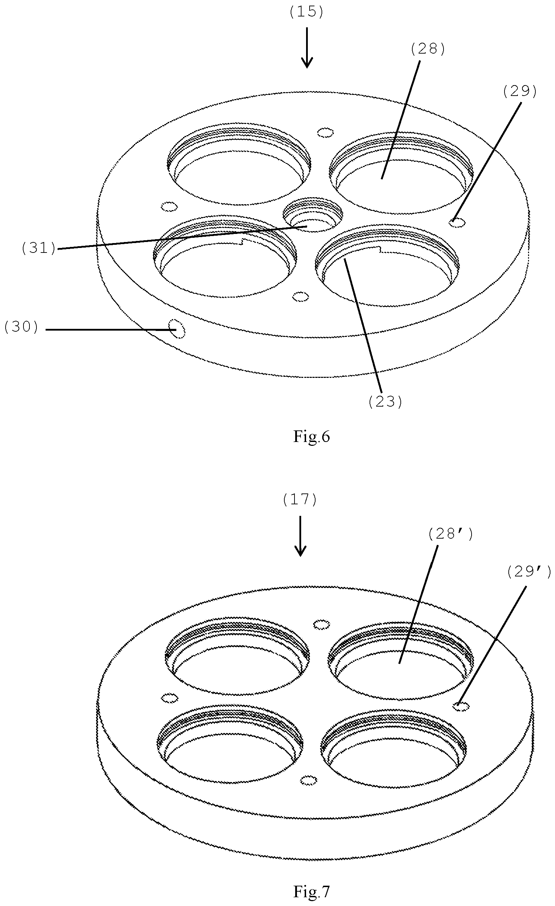

In a preferred embodiment of the present invention the interconnected base (15), represented in the figure 6, comprises:

- at least a base cavity (28), for fitting the respective vertical vessel (12);
- at least a base cavity for support (29), for fitting the respective vertical support shaft (14);
- a horizontal leak hole (30), represented in the figure 2, located at the side face of the base (15), to empty the sump feed (23);
- a base central cavity (31), for fitting a vertical and central feed vessel (13); and
- the sump feed (23) located immediately under the base central cavity (31) and also under each of the respective vertical vessel (12).

In a preferred embodiment of the present invention, the base (17), represented in the figure 7, comprises:

- at least a base cavity (28'), for fitting the respective vertical vessel (12'); and
- at least a base cavity for support (29'), for fitting the respective vertical support shaft (14').

In a preferred embodiment of the present invention the interconnected base (15), represented in the figure 6, of the reactor for biofilm formation (3) has the base cavities (28) in equal number to the number of the vertical vessels (12).

In a preferred embodiment of the present invention, the system for rotating (10) comprises a motor, not represented in the figures, to rotate at least one vertical shaft (8), not represented in the figures, wherein the motor rotates synchronously each of the vertical shafts (8).

Furthermore, the system for rotating (10) comprises:

- a pulley (32), represented in the figure 1, fixed in the vertical shaft (8);
- a pulley stretcher (33), represented in the figure 1; and
- a synchronizing belt, not represented in the figures, that interconnects at least a vertical shaft (8), preferably four vertical shafts (8), for rotating synchronously each of the vertical shafts (8) by means of the motor.

Head (1)

A head (1), represented in the figures 1 and 8, for a reactor for biofilm formation (3) , comprises a principal cover (6) that includes at least a vertical hole (7), wherein it is inserted a vertical shaft (8) into each vertical hole (7) and each vertical shaft (8) comprises at least a fixed cylinder (9).

Furthermore, the head (1) comprises a system for rotating (10) each respective vertical shaft (8) around its own axis.

As represented in figure 1, of the present invention, the vertical shaft (8) comprises cylinders (9) which are equally distant from each other and arranged in series.

In one of several embodiments of the present invention, the cylinder (9), as represented in the figure 1, comprises a cylindrical shape, a sampling surface including:

- a diameter of about 21 mm;
- a length of about 50 mm; and
- a surface area of about 33 cm$^2$.
- a longitudinal hole (27), for insertion into the vertical shaft (8); and
- a fixation section including:
- a diameter of about 18 mm; and
- a length of about 10 mm for cylinder (9) fixation and removal in the vertical shaft (8).

In one of several embodiments of the present invention, the cylinder (9) may have other different measurements for the sampling surface and the fixation section than the measurements mention to in the previous paragraph.

In one of several embodiments of the present invention, the use of a cylinder (9) with a higher surface area increases the sampling area, improving the homogeneity of biofilm and decreasing the effects of changes in flow patterns in the boundaries of each cylinder, a phenomenon commonly observed in alternative reactors.

It is possible to form biofilms uniformly, independent of the cylinder (9) position.

In one of several embodiments of the present invention, different materials can be used as substratum for biofilm formation.

In one of several embodiments of the present invention, the cylinder (9) may be made of only one material or different materials simultaneously, such as:

- metals;
- metallic alloys;
- plastics;
- glass; and
- coatings and surface coats.

In one of several embodiments of the present invention, the coatings and surface coats, above mentioned in the previous paragraph, comprise a nanomaterial or a surface chemically modified.

In one of several embodiments of the present invention, materials may be selected according to the objective of the work and may be metals and metallic alloys, plastics, glass and also modified materials and surfaces.

Reactor for biofilm formation (3)

As can be seen in the respective figures 2 and 3, the present invention comprises a reactor for biofilm formation (3).

In the figures 1 and 8 is represented a head (1), which is common to the reactor for biofilm formation (3).

In one of several embodiments of the present invention, the reactor for biofilm formation (3), represented in the figure 2, comprises:

- the head (1) that is inserted into, and
- a platform for biofilm formation (2), which are respectively shaped so as to form an apparatus, namely a reactor for biofilm formation (3) that operates:
- under batch;
- fed batch; or
- continuous operating conditions.

In an embodiment outside the subject-matter of the claims but useful for understanding the disclosure, the reactor for biofilm inactivation and removal from the adhesion surface (5), represented in the figure 3, comprises:

- the head (1) that is inserted into, and
- a platform for biofilm inactivation and removal from the adhesion surface (4), which are respectively shaped so as to form an apparatus, namely a reactor for biofilm inactivation and removal from the adhesion surface (5) that operates:
- under batch operating condition.

The platform for biofilm formation (2), represented in the figure 2, comprises:

- a feed and drainage cover (11);
- at least a vertical vessel (12);
- a vertical and central feed vessel (13);
- at least a vertical support shaft (14); and
- an interconnected base (15).

In one of several embodiments of the present invention, the reactor for biofilm formation (3), as represented in the figures 2 and 3, comprise at least a vertical support shaft (14).
In one of several embodiments of the present invention, the reactor for biofilm formation (3), as represented in the figures 2 and 3, according to the one of the respective embodiments of the invention, comprise preferably four vertical support shaft (14).
In one of several embodiments of the present invention, according to the one of the respective embodiments of the invention, the vertical support shaft (14) is manufactured in aluminium and stainless steel.
In one of several embodiments of the present invention, the system for rotating (10), not represented in the figures, comprises a motor, to rotate at least a vertical shaft (8).
In one of several embodiments of the present invention, the motor synchronously rotates each of the vertical shaft (8).
In one of several embodiments of the present invention, the system for rotating (10) each vertical shaft (8) comprises:

- a pulley (32) fixed in the vertical shaft (8);
- a pulley stretcher (33); and
- a synchronizing belt that interconnects the four vertical shafts (8) for rotating synchronously each of the vertical shafts (8) by means of the motor.

According to the present invention, the feed and drainage cover (11) comprises a system for feeding and draining, not represented in the figures, which includes:

- an external pump, for feeding through an external tube the vertical and central feed vessel (13); and
- at least a horizontal drainage hole (22), for draining each of the vertical vessels (12).

As represented in the figures 1, 2 and 3, in one of several embodiments of the present invention, the vertical shaft (8) is held in the principal cover (6) and suspended inside the vessel (12).
In an embodiment outside the subject-matter of the claims but useful for understanding the disclosure, , the platform for biofilm inactivation and removal from the adhesion surface (4), represented in the figure 3, comprises:

- a cover (16);
- at least a vertical vessel (12');
- at least a vertical support shaft (14'); and
- a base (17).

According to the present invention, the feed and drainage cover (11), represented in the figures 2, 4 and 8, comprises:

- at least a vertical insertion hole (18), for insertion of each respective vertical vessel (12);
- a vertical feed central hole (19), for insertion of the respective vertical and central feed vessel (13);
- at least a vertical cavity (20), located at the bottom of the feed and drainage cover (11), for fitting the respective vertical vessel (12);
- at least a vertical hole for support (21) for insertion of each respective vertical support shaft (14); and
- at least a horizontal drainage hole (22), located at the side face of the feed and drainage cover (11), for drainage of the respective vertical vessel (12).

The cover (16), represented in the figures 3, 5 and 9, comprises:

- at least a vertical insertion hole (18'), for insertion of each respective vertical vessel (12');
- at least a vertical cavity (20'), located at the bottom of the cover (16), for fitting the respective vertical vessel (12'); and
- at least a vertical hole for support (21'), for insertion of each respective vertical support shaft (14').

In one of several embodiments of the present invention as represented in the figures 4 and 8, in the feed and drainage cover (11), the number of:

- the vertical insertion holes (18);
- the vertical cavities (20); and
- horizontal drainage holes (22);

is equal to the number of the vertical vessels (12), represented in the figure 2.

As represented in the figure 5 and 9, in the cover (16), the number of the vertical insertion holes (18') and the vertical cavities (20') is equal to the number of the vertical vessels (12'), as represented in the figures 3 and 9.

The reactor according to the present invention may be constructed in transparent materials, allowing a proper observation of the biofilm formation and removal.

In one of several embodiments of the present invention, the feed and drainage cover (11) and cover (16) are manufactured with a translucent material, such as a polymethyl methacrylate or glass.

In one of several embodiments of the present invention, the interconnected base (15) and the base (17) are manufactured with a translucent material, such as polymethyl methacrylate or glass.

As represented in the figure 6, each of the vertical vessels (12) of the reactor for biofilm formation (3) are interconnected through the interconnected base (15) that comprises a sump feed (23).

In an embodiment outside the subject-matter of the claims but useful for understanding the disclosure, as represented in the figures 3 and 9, in one of several embodiments of the present invention, each of the vertical vessels (12') of the reactor for biofilm inactivation and removal from the adhesion surface (5) are independent from one another.

In one of several embodiments of the present invention, when the reactor for biofilm formation (3) operates under batch operating condition, a bacterial culture (24) and an initial nutrient medium (25) are fed to the said reactor for biofilm formation (3).

In one of several embodiments of the present invention, when the reactor for biofilm formation (3) operates under fed batch or continuous operating conditions, its operation comprises feeding:

- a bacterial culture (24);
- an initial nutrient medium (25); and
- a diluted nutrient medium (26).

In an embodiment outside the subject-matter of the claims but useful for understanding the disclosure, the reactor for biofilm inactivation and removal from the adhesion surface (5) comprises feeding a chemical agent (34) and a neutralizer solution (35).

The reactor for biofilm formation (3) of the present invention is adapted to carry out assays with the same initial nutrient medium (25) or diluted nutrient medium (26), using the interconnected base (15). Alternatively, it is possible to perform simultaneous assays with different initial nutrient media (25) using the base (17) only under batch operating condition.

As it will be understood by a person skilled in the art, each vertical shaft (8) may be configured to rotate in a specific rotational speed. Therefore, it is possible to perform simultaneous assays in both reactor for biofilm formation (3) or reactor for biofilm inactivation and removal from the adhesion surface (5) employing different rotational speeds.

In an embodiment outside the subject-matter of the claims but useful for understanding the disclosure, in the case of the reactor for biofilm inactivation and removal from the adhesion surface (5), a base (17) composed by, for example, four independent vessels may be used to apply different chemical treatments simultaneously, with at least two replicates for each condition.

The reactor for biofilm formation (3) overcomes the limitations of prior art technologies and their main advantages are:

- The reactors may be compact and require a lower workspace or a lower ratio volume of reagents/work volume, e. g. 2.5 L;
- The cylinders have high sampling area (e. g. 33 cm$^2$ for each cylinder having a diameter of 2.1 cm and a length of 5.0 cm). The use of a cylinder with high surface area (sampling area) improves the homogeneity of biofilm, decreases the effects of changes in flow patterns in the boundaries of each cylinder, which is a phenomenon commonly observed in prior state of the art reactors having small or flat coupons;
- It is possible to form biofilms uniformly independently of a cylinder position;
- The cylinders can be made of different materials as a substratum for biofilm formation. The reactors may be used with cylinders made of only one material or different materials simultaneously;
- The control of hydrodynamic conditions is very simple, only depending on the adjustment of the cylinders rotational speed through, for example, an overhead stirrer and a synchronized belt that connects all the cylinders;
- The use of cylindrical surfaces for biofilm sampling increases the similarity with main of the industrial pipes where biofilms are formed;
- The reactors are adequate to study biofilm formation and its control by different chemical and/or mechanical treatments. Useful to mimic pipe flushing as a mechanical treatment;
- No coupons are used and changes in flow pattern are reduced;
- The different vessels, where cylinders are inserted, avoid cross flow;
- The cylinders are easily removed from the respective vertical shaft. Each cylinder could easily be removed one by one from the respective vertical shaft;
- As illustrated in the drawings, the reactor is composed by different vessels that contain several cylinders each. The number of sampling cylinders may be increased up to 4 times and the use of interconnected vessels allows the biofilm formation under the same conditions without hydrodynamic interference due to cylinders rotation.

Data relating the rotational speed (in rotation per minute - rpm) with the Reynolds number (Re - dimensionless) and shear stress (in Pascal - Pa) on the cylinder (9) surface are provided in the table 1.

Table 1: rotational speed and corresponding Reynolds number and shear stress

| Rotational speed (rpm) | Reynolds number (Re) | Shear stress (Pa) |
| --- | --- | --- |
| 50 | 578 | 0.04 |
| 100 | 1155 | 0.12 |
| 150 | 1733 | 0.23 |
| 200 | 2310 | 0.37 |
| 250 | 2888 | 0.55 |
| 300 | 3465 | 0.75 |
| 350 | 4043 | 0.97 |
| 400 | 4620 | 1.22 |
| 450 | 5198 | 1.49 |
| 500 | 5775 | 1.78 |
| 750 | 8663 | 3.54 |
| 1000 | 11550 | 5.77 |
| 1250 | 14438 | 8.43 |
| 1500 | 17325 | 11.50 |
| 1750 | 20213 | 14.94 |

(continued)

| Rotational speed (rpm) | Reynolds number (Re) | Shear stress (Pa) |
|---|---|---|
| 2000 | 23100 | 18.75 |

As it will fully to be understood by a person skilled in the art, when the Reynolds number is relatively small, the biofilm formed has a less cohesive structure, which can be more easily destroyed. In an opposite direction, under a higher Reynolds number, the biofilm formed is more adapted to the stressful conditions, having a higher cohesion, making its inactivation and removal more difficult.

The calculation of rotational Reynolds number (Re) is based on the annulus gap between cylinder surface and the vessel wall:

$$Re = \frac{a(b-a)\rho N}{\mu}$$

where a is the cylinder diameter, b is the inner diameter of the vessel, $\rho$ is the density of nutrient media, N is the rotational speed and $\mu$ is the viscosity of nutrient medium. The shear stress for a rotating cylinder is given by:

$$\tau = \frac{f\rho v^2}{2}$$

where f is the Fanning factor and v is the fluid velocity in the cylinder surface.
The Fanning factor (f) for a rotating cylinder is calculated according to:

$$f = 0.158 Re^{-0.3}$$

The velocity of fluid in the cylinder surface is given by:

$$v = N\pi a$$

Process for biofilm formation and process for biofilm inactivation and removal from the adhesion surface (not part of the invention)

Disclosed herein is also an integrated process that comprises two processes, concretely the first one, the process for biofilm formation, and the second one, the process for biofilm inactivation and removal from the adhesion surface, which comprises the following steps:

i) biofilm formation by using the reactor for biofilm formation (3);
ii) removal of the respective head (1) from the platform for biofilm formation (2);
iii) insertion of the same head (1), removed from the platform for biofilm formation (2) in step ii), into each of the respective vertical vessel (12'), which constitute the platform for biofilm inactivation and removal from the adhesion surface (4); and
iv) biofilm inactivation and removal from the adhesion surface by the reactor for biofilm inactivation and removal from the adhesion surface (5) by using the reactor for biofilm inactivation and removal from the adhesion surface (5).

Process for biofilm formation

The present invention refers to a process for biofilm formation, by means of the reactor for biofilm formation (3), which comprises the following steps:

i) cleaning and disinfecting the cylinders (9) and the vertical vessels (12); and
ii) biofilm formation by using the reactor for biofilm formation (3), wherein:

- the step of ii) biofilm formation can be performed under batch operating condition; or
- the step of ii) biofilm formation can be performed under fed batch operating condition; or

- the step of ii) biofilm formation can be performed under continuous operating condition.

In one of several embodiments of the present invention, the step i) cleaning and disinfecting the cylinders (9) and the vertical vessels (12), comprises the following substeps:

i.i) immerse the cylinder (9) in a trough with a solution of commercial detergent;
i.ii) rinse the cylinder (9) in a trough with sterile distilled water and then immerse it in 70% ethanol (v/v) for about 5 min;
i.iii) rinse the cylinder (9) in a trough for three times with sterile distilled water and dry it at room temperature;
i.iv) insert at least one cylinder (9) into the respective vertical shaft (8);
i.v) insert the head (1) into each of the respective vertical vessel (12) which constitutes the platform for biofilm formation (2), and which is a part of the reactor for biofilm formation (3);
i.vi) fill the vertical vessels (12) through the vertical and central feed vessel (13) with about 10% bleach solution (v/v) for about 30 min;
i.vii) remove about 10% bleach solution (v/v) through the horizontal leak hole (30);
i.viii) fill the vertical vessel (12) through the vertical and central feed vessel (13) with sterile distilled water; and
i.ix) remove the sterile distilled water through the horizontal leak hole (30).

In a preferred embodiment of the present invention, the step of ii) biofilm formation, when the reactor for biofilm formation (3) operates under batch operating conditions, comprises the following substeps:

ii.i) fill the vertical vessel (12), by the vertical and central feed vessel (13), with the bacterial culture (24);
ii.ii) fill the vertical vessel (12) by the vertical and central feed vessel (13), with the initial nutrient medium (25); and
ii.iii) rotate at least one cylinder (9) around its own axis, during about 24 h to several months and with a rotation from 0 to about 2000 rpm.

In a preferred embodiment of the present invention, the step of ii) biofilm formation, when the reactor for biofilm formation (3) operates under fed batch operating conditions, comprises the following substeps:

ii.i) fill the vertical vessel (12), by the vertical and central feed vessel (13), with a bacterial culture (24);
ii.ii) fill the vertical vessel (12), by the vertical and central feed vessel (13), with the initial nutrient medium (25);
ii.iii) feed periodically the vertical vessel (12), by means of the external pump (29) through an external tube which is directly linked to the vertical and central feed vessel (13), with sterile diluted nutrient medium (26); and
ii.iv) rotate at least one cylinder (9) around its own axis during about 24 h to several months and with a rotation from 0 to about 2000 rpm.

In a preferred embodiment of the present invention, the step of ii) biofilm formation, when the reactor for biofilm formation (3) operates in continuous operating conditions, comprises the following substeps:

ii.i) fill the vertical vessel (12), by the vertical and central feed vessel (13), with a bacterial culture (24);
ii.ii) fill the vertical vessel (12), by the vertical and central feed vessel (13), with the initial nutrient medium (25);
ii.iii) continuously feed the vertical vessel (12), by means of the external pump (29) through a tube which is directly linked to vertical and central feed vessel (13), with sterile diluted nutrient medium (26);
ii.iv) drain the reactor for biofilm formation (3) through the top horizontal drainage hole (22); and
ii.v) rotate at least one cylinder (9) around its own axis, during about 24 h to several months and with a rotation from 0 to about 2000 rpm.

In one of the several embodiments of the present invention, the bacterial culture (24) used for biofilm formation comprises the bacterium, carbon, nitrogen and phosphorous sources.
In one of several embodiments of the present invention, the initial nutrient medium (25) used for biofilm formation comprises carbon, nitrogen and phosphorous sources.
In one of several embodiments of the present invention, the sterile diluted nutrient medium (26), used for biofilm formation comprises 100-fold diluted carbon, nitrogen and phosphorous sources from the initial nutrient medium (25) in adequate buffer, for example, phosphate buffer.
The table 2 describes the possible composition of the initial nutrient medium (25) and of the feeding medium for biofilm formation by Bacillus cereus or/and Escherichia coli or/and Pseudomonas fluorescens or/and Staphylococcus aureus (single- or mixed-biofilms).

Table 2: composition of the initial nutrient medium (25) and of the feeding medium for biofilm formation by *Bacillus cereus* or/and *Escherichia coli* or/and *Pseudomonas fluorescens* or/and *Staphylococcus aureus* (single- or mixed-biofilms)

| Compounds | Initial concentration (g/L) | Fed concentration (g/L) |
|---|---|---|
| Peptone | 0.0 - 2.5 | 0.0 - 0.025 |
| Yeast extract | 0.0 - 5.0 | 0.0 - 0.05 |
| Glucose | 0.0 - 5.0 | 0.0 - 0.05 |
| Tryptone | 0.0 - 17.0 | 0.0 - 0.17 |
| Starch | 0.0 - 1.5 | 0.0 - 0.015 |
| Casein hydrolysate | 0.0 - 17.5 | 0.0 - 0.175 |
| Soy | 0.0 - 3.0 | 0.0 - 0.03 |
| Beef infusion solids | 0.0 - 2.0 | 0.0 - 0.02 |
| Sodium pyruvate | 0.0 - 0.3 | 0.0 - 0.003 |
| Sodium chloride | 0.0 - 8.5 | 0.0 - 8.5 |
| Dipotassium phosphate | 0.0 - 2.5 | 0.0 - 0.025 |
| Magnesium sulphate | 0.0 - 0.1 | 0.0 - 0.001 |
| Potassium phosphate monobasic | 0.0 - 1.9 | 0.0 - 1.9 |
| Sodium phosphate dibasic | 0.0 - 2.6 | 0.0 - 2.6 |

Process for biofilm inactivation and removal from the adhesion surface (not according to the invention)

Disclosed herein is further a process for biofilm inactivation and removal from the adhesion surface, by the means of the reactor for biofilm inactivation and removal from the adhesion surface (5), which comprises the following steps:

i) fill each of the vertical vessel (12') through the respective upper opening which constitutes the platform for biofilm inactivation and removal from the adhesion surface (4) with a chemical agent (34) wherein each of the vertical vessel (12') is fill with the same chemical agent (34); or a different chemical agents (34);

ii) remove the head (1) from the platform for biofilm formation (2);

iii) insert the same head (1), removed from the platform for biofilm formation (2) in step ii), into each of the respective vertical vessel (12') which constitutes the platform for biofilm inactivation and removal from the adhesion surface (4);

iv) rotate at least one cylinder (9) around its own axis, immersed in the chemical agent (34), during about 30 seconds to about two hours and with a rotation 10 to about 2000 rpm, according to the desired application;

v) remove the head (1) from the reactor for biofilm inactivation and removal from the adhesion surface (5);

vi) remove the chemical agent (34) through the respective upper opening which constitutes the platform for biofilm inactivation and removal from the adhesion surface (4);

vii) fill the vertical vessel (12') through the respective upper opening with a neutralizer solution (35) depending on the chemical agent (34) used;

viii) insert the head (1), removed from the reactor for biofilm inactivation and removal from the adhesion surface (5) in step v), into each of the respective vertical vessel (12') which constitutes the same platform for biofilm inactivation and removal from the adhesion surface (4); and

ix) rotate at least one cylinder (9) around its own axis, during about 1 to about 30 minutes and with a rotation of about 10 to about 2000 rpm, according to the desired application.

In one of several embodiments of the present invention, the step i) each of the vertical vessel (12) is fill with the same chemical agent (34) or different chemical agents (34).

An embodiment, , the chemical agent (34), for biofilm inactivation and removal from the adhesion surface, is for example a disinfectant solution, and may be selected from one or more of the groups: aldehydes (e. g. glutaraldehyde, ortho-phthalaldehyde); peroxides (e. g. hydrogen peroxide, peracetic acid); halogens (e. g.bleach at a maximum of 15% (v/v), sodium hypochlorite; chlorine dioxide; iodine); biguanides; quaternary ammonium compounds; phenolics; phytochemicals; organic acids; inorganic acids; anionic surfactants; alcohols; and nanoparticles;

Biofilm formation, biofilm inactivation and removal from the adhesion surface

Biofilm is formed on the surface of each cylinder (9), in the reactor for biofilm formation (3), which comprises:

- the bacterial culture (24);
- the initial nutrient medium (25); and
- the diluted nutrient medium (26).

An embodiment useful for understanding the invention, biofilm inactivation and removal from the adhesion surface comprises chemical agent (34), neutralizer solution (35), and inactivation and removal from the adhesion surface by reactor for biofilm inactivation and removal from the adhesion surface (5).

The subject matter described above is provided as an illustration of the present invention and should not be construed as limiting it. The scope of the invention is defined by the annexed claims.

**Claims**

1.  A reactor for biofilm formation (3) comprising a head (1) for a reactor for biofilm formation (3), comprising:

    a principal cover (6), wherein the principal cover (6) comprises:

    a plurality of vertical holes (7), wherein it is inserted a vertical shaft (8) into each one of the vertical holes (7) wherein each vertical shaft (8) comprises a plurality of fixed cylinders (9), wherein the cylinders (9) are arranged in series; and the head (1) further comprising:

    a system for rotating (10) each respective vertical shaft (8) around its own axis;
    wherein the head (1) is inserted into a platform for biofilm formation (2), wherein the platform for biofilm formation (2) comprises:

    - a feed and drainage cover (11);
    - a plurality of vertical vessels (12), preferably four vertical vessels (12);
    - a vertical and central feed vessel (13);
    - a plurality of vertical support shafts (14), preferably four vertical support shafts (14); and
    - an interconnected base (15);

    and the vertical shaft (8) being:

    - held in the principal cover (6); and
    - suspended inside the vessel (12);

    wherein the feed and drainage cover (11) comprises:

    - a plurality of vertical insertion holes (18), for insertion of each four respective vertical vessel (12);
    - a vertical feed central hole (19), for insertion of the respective vertical and central feed vessel (13);
    - a plurality of vertical cavities (20), located at the bottom of the feed and drainage cover (11), for fitting the respective vertical vessel (12);
    - a plurality of vertical holes for support (21), for insertion of each respective four vertical support shafts (14); and
    - a plurality of horizontal drainage holes (22), located at the side face of the feed and drainage cover (11), for drainage of the respective vertical vessel (12);

    and the feed and drainage cover (11) further comprising a system for feeding and draining that includes an external pump, for feeding the vertical and central feed vessel (13) through an external tube.

2.  The reactor for biofilm formation (3), according to claim 1, wherein the vertical vessel (12) is tight and comprises:

    - a tubular shape; and
    - a working volume including:

- a diameter of about 27 to about 108 mm; and
- a length of about 136 to about 544 mm,

    and each vertical vessel (12) being interconnected through the interconnected base (15) that comprises a sump feed (23);
    and each vertical vessel (12) is fed by the vertical and central feed vessel (13), which is interconnected with all the vertical vessels (12) through the sump feed (23) of the interconnected base (15).

3. The reactor for biofilm formation (3), according to any of the claims 1 or 2, wherein the cylinders (9) comprise:

    - a cylindrical shape;
    - a sampling surface;
    - a longitudinal hole (27), for insertion into the vertical shaft (8); and
    - a fixation section,

and the cylinders (9) being manufactured using a material selected from the list consisting of:

    - metals;
    - metallic alloys;
    - plastics;
    - glass; and
    - coatings and surface coats, wherein the coatings and surface coats comprising a nanomaterial or a chemically modified surface;

and the cylinders (9) are fixed in the vertical shaft (8); and the cylinders (9) rotate around its own axis.

4. The reactor for biofilm formation (3), according to any of the claims 1 or 2 or 3, wherein the interconnected base (15) comprises:

    - a plurality of base cavities (28), for fitting the respective vertical vessel (12);
    - a plurality of base cavities for support (29), for fitting the respective vertical support shaft (14);
    - a horizontal leak hole (30), located at side face of the base (15), for empty the sump feed (23);
    - a base central cavity (31), for fitting a vertical and central feed vessel (13); and
    - the sump feed (23) located immediately under the base central cavity (31) and also under each of the respective vertical vessel (12).

5. The reactor for biofilm formation (3) according to the claim 4, wherein the interconnected base (15) of the reactor for biofilm formation (3) comprises base cavities (28) in equal number to the number of the vertical vessels (12).

6. The reactor for biofilm formation (3), according to any of the claims 1 or 2 or 3 or 4 or 5, wherein the system for rotating (10) comprises a motor, to rotate a plurality of vertical shafts (8), wherein the motor rotates synchronously each of the vertical shafts (8),
and the system for rotating (10) further comprises:

    - a pulley (32) fixed in the vertical shaft (8);
    - a pulley stretcher (33); and
    - a synchronizing belt that interconnects the four vertical shafts (8) for rotating synchronously each of the vertical shafts (8) by means of the motor.

7. A process for biofilm formation, **characterized by** using the reactor for biofilm formation (3) of any of the claims 1 or 2 or 3 or 4 or 5 or 6 and comprising the following steps:

    i) cleaning and disinfecting the reactor for biofilm formation using a chemical agent (3); and
    ii) biofilm formation by using the reactor for biofilm formation (3), wherein:

        - the step of ii) biofilm formation is performed under batch operating condition; or
        - the step of ii) biofilm formation is performed under fed batch operating condition; or
        - the step of ii) biofilm formation is performed under continuous operating condition.

8. The process for biofilm formation according to claim 7, wherein the step of ii) biofilm formation comprises the following substeps:

ii.i) fill the vertical vessels (12), by the vertical and central feed vessel (13), with the bacterial culture (24);
ii.ii) fill the vertical vessels (12) by the vertical and central feed vessel (13), with the initial nutrient medium (25);
ii.iii) for fed batch or continuous operating conditions, feed the vertical vessels (12), by means of the external pump (29) through an external tube which is directly linked to the vertical and central feed vessel (13), with sterile diluted nutrient medium (26); and
ii.iv) rotate the cylinders (9) around their own axis, during about 24 h to several months and with a rotation from 0 to about 2000 rpm.

**Patentansprüche**

1. Ein Reaktor zur Biofilmbildung (3), umfassend einen Kopf (1) für einen Reaktor zur Biofilmbildung (3), umfassend:

einen Hauptdeckel (6), wobei der Hauptdeckel (6) umfasst:

eine Vielzahl vertikaler Öffnungen (7), wobei in jede der vertikalen Öffnungen (7) eine vertikale Welle (8) eingesetzt ist
wobei jede vertikale Welle (8) eine Vielzahl feststehender Zylinder (9) umfasst, wobei die Zylinder (9) in Reihe angeordnet sind; und der Kopf (1) ferner umfasst:

ein System zum Drehen (10) jeder einzelnen vertikalen Welle (8) um ihre eigene Achse;
wobei der Kopf (1) in eine Plattform zur Biofilmbildung (2) eingesetzt ist, wobei die Plattform zur Biofilmbildung (2) umfasst:

- einen Zuführ- und Abflussdeckel (11);
- eine Vielzahl vertikaler Behälter (12), bevorzugt vier vertikale Behälter (12);
- einen vertikalen und zentralen Zuführbehälter (13);
- eine Vielzahl vertikaler Stützwellen (14), bevorzugt vier vertikale Stützwellen (14); und
- eine verbundene Grundplatte (15);

und die vertikale Welle (8):

- in dem Hauptdeckel (6) gehalten wird; und
- im Inneren des Behälters (12) aufgehängt ist;

wobei der Zuführ- und Abflussdeckel (11) umfasst:

- eine Vielzahl vertikaler Einführungsöffnungen (18) zum Einführen von jeweils vier entsprechenden vertikalen Behältern (12);
- eine zentrale Zuführöffnung (19) zum Einführen des entsprechenden vertikalen und zentralen Zuführbehälters (13);
- eine Vielzahl vertikaler Hohlräume (20), die sich am Boden des Zuführ- und Abflussdeckels (11) befinden, zur Aufnahme des entsprechenden vertikalen Behälters (12);
- eine Vielzahl vertikaler Stützöffnungen (21) zum Einsetzen der jeweils entsprechenden vier vertikalen Stützwellen (14); und
- eine Vielzahl horizontaler Abflussöffnungen (22), die sich an der Seitenfläche des Zuführ- und Abflussdeckels (11) befinden, um den entsprechenden vertikalen Behälter (12) zu entleeren;

und den Zuführ- und Abflussdeckel (11), ferner umfassend ein System für die Zufuhr und den Abfluss, das eine externe Pumpe für die Versorgung des vertikalen und zentralen Zuführbehälters (13) durch einen externen Schlauch umfasst.

2. Der Reaktor zur Biofilmbildung (3) nach Anspruch 1, wobei der vertikale Behälter (12) dicht ist und umfasst:

- eine röhrenförmige Form; und

- ein Arbeitsvolumen, umfassend:
- einen Durchmesser von etwa 27 bis etwa 108 mm; und
- eine Länge von etwa 136 bis etwa 544 mm,

und jeder vertikaler Behälter (12) durch die verbundene Grundplatte (15) verbunden ist, die eine Sumpf-Zuführung (23) umfasst;
und jeder vertikale Behälter (12) durch den vertikalen und zentralen Zuführbehälter (13) versorgt wird, der mit allen vertikalen Behältern (12) durch die Sumpf-Zuführung (23) der verbundenen Grundplatte (15) verbunden ist.

3. Der Reaktor zur Biofilmbildung (3) nach einem der Ansprüche 1 oder 2, wobei die Zylinder (9) umfassen:

- eine zylindrische Form;
- eine Entnahmeoberfläche;
- eine Längsöffnung (27) zum Einführen in die vertikale Welle (8); und
- einen Fixierungsabschnitt,
und die Zylinder (9) aus einem Werkstoff hergestellt sind, der aus der nachstehenden Liste ausgewählt ist, bestehend aus:

- Metallen;
- Metalllegierungen;
- Kunststoffen;
- Glas; und
- Beschichtungen und Oberflächenschichten, wobei die Beschichtungen und Oberflächenschichten ein Nanomaterial oder eine chemisch modifizierte Oberfläche umfassen;

und die Zylinder (9) in der vertikalen Welle (8) befestigt sind; und die Zylinder (9) sich um ihre eigene Achse drehen.

4. Der Reaktor zur Biofilmbildung (3) nach einem der Ansprüche 1 oder 2 oder 3, wobei die verbundene Grundplatte (15) umfasst:

- eine Vielzahl von Hohlräumen in der Bodenplatte (28) zur Aufnahme des entsprechenden vertikalen Behälters (12);
- eine Vielzahl von Hohlräumen in der Bodenplatte als Abstützung (29), in die die entsprechende vertikale Stützwelle (14) eingesetzt wird;
- eine horizontale Lecköffnung (30), die sich an der Seitenfläche der Bodenplatte (15) befindet, um die Sumpf-Zuführung (23) zu entleeren;
- einen zentralen Hohlraum in der Bodenplatte (31) zur Aufnahme eines vertikalen und zentralen Zuführbehälters (13); und
- die Sumpf-Zuführung (23) sich unmittelbar unter dem zentralen Hohlraum in der Bodenplatte (31) befindet und auch unter jedem der entsprechenden vertikalen Behälter (12).

5. Der Reaktor zur Biofilmbildung (3) nach Anspruch 4, wobei die verbundene Grundplatte (15) des Reaktors zur Biofilmbildung (3) Hohlräume in der Bodenplatte (28) in einer Anzahl aufweist, die der Anzahl der vertikalen Behälter (12) entspricht.

6. Der Reaktor zur Biofilmbildung (3) nach einem der Ansprüche 1 oder 2 oder 3 oder 4 oder 5, wobei das System zum Drehen (10) einen Motor umfasst, um eine Vielzahl vertikaler Wellen (8) zu drehen, wobei der Motor jede der vertikalen Wellen (8) synchron dreht, und das System zum Drehen (10) ferner umfasst:

- eine Riemenscheibe (32), die an der vertikalen Welle (8) befestigt ist;
- einen Riemenspanner (33); und
- einen Synchronriemen, der die vier vertikalen Wellen (8) miteinander verbindet, um jede der vertikalen Wellen (8) mit Hilfe des Motors synchron zu drehen.

7. Ein Verfahren zur Biofilmbildung, **gekennzeichnet durch** die Verwendung des Reaktors zur Biofilmbildung (3) nach einem der Ansprüche 1 oder 2 oder 3 oder 4 oder 5 oder 6 und umfassend die folgenden Schritte:

i) Reinigen und Desinfizieren des Reaktors zur Biofilmbildung unter Verwendung eines chemischen Mittels (3); und

ii) Biofilmbildung durch Verwendung des Reaktors zur Biofilmbildung (3), wobei:

- der Schritt ii) der Biofilmbildung unter Bedingungen für den Batchbetrieb durchgeführt wird; oder
- der Schritt ii) der Biofilmbildung unter Bedingung für den Fed-Batchbetrieb durchgeführt wird; oder
- der Schritt ii) der Biofilmbildung unter kontinuierlichen Betriebsbedingungen durchgeführt wird.

8. Das Verfahren zur Biofilmbildung nach Anspruch 7, wobei der Schritt ii) der Biofilmbildung die folgenden Teilschritte umfasst:

ii.i) Befüllen der vertikalen Behälter (12) durch den vertikalen und zentralen Zuführbehälter (13) mit der Bakterienkultur (24);

ii.ii) Befüllen der vertikalen Behälter (12) über den vertikalen und zentralen Zuführbehälter (13) mit dem Anfangsnährmedium (25);

ii.iii) Versorgen der vertikalen Behälter (12) mit sterilem verdünntem Nährmedium (26) mit Hilfe der externen Pumpe (29) über einen externen Schlauch, der direkt mit dem vertikalen und zentralen Zuführbehälter (13) verbunden ist, wenn die Anlage im Fed-Batch- oder Dauerbetrieb betrieben wird; und

ii.iv) Drehen der Zylinder (9) über einen Zeitraum von etwa 24 h bis zu mehreren Monaten und mit einer Drehzahl von 0 bis etwa 2000 U/min um ihre eigene Achse.

**Revendications**

1. Un réacteur pour la formation de biofilms (3) comprenant une tête (1) pour un réacteur pour la formation de biofilms (3), comprenant :

une couverture principale (6), dans laquelle la couverture principale (6) comprend :

une pluralité de trous verticaux (7), dans lesquels est inséré un arbre vertical (8) dans chacun des trous verticaux (7)

dans lequel chaque arbre vertical (8) comprend une pluralité de cylindres fixes (9), dans lequel les cylindres (9) sont arrangés en séries ; et la tête (1) comprend également :

un système pour pivoter (10) chaque arbre vertical respectif (8) autour de son propre axe ; dans lequel la tête (1) est insérée dans une plateforme pour la formation de biofilms (2), dans lequel la plateforme pour la formation de biofilms (2) comprend :

- une couverture d'alimentation et de drainage (11) ;
- une pluralité de récipients verticaux (12), préférablement quatre récipients verticaux (12) ;
- un récipient d'alimentation vertical et central (13) ;
- une pluralité d'arbres de support verticaux (14), préférablement quatre arbres de support verticaux (14) ; et
- une base interconnectée (15) ;

et l'arbre vertical (8) étant :

- placé dans la couverture principale (6) ; et
- suspendu à l'intérieur du récipient (12) ;

dans lequel la couverture d'alimentation et de drainage (11) comprend :

- une pluralité de trous d'insertion verticaux (18), pour insérer chacun des quatre récipients verticaux respectifs (12) ;
- un trou central d'alimentation verticale (19), pour insérer le récipient d'alimentation vertical et central respectif (13) ;
- une pluralité de cavités verticales (20), situées au fond de la couverture d'alimentation et de drainage (11), pour fixer le récipient vertical respectif (12) ;

- une pluralité de trous verticaux servant de support (21), pour l'insertion de chacun des arbres de support verticaux respectifs (14) ; et
- une pluralité de trous de drainage horizontaux (22), situés sur la face latérale de la couverture d'alimentation et de drainage (11), pour le drainage du récipient vertical respectif (12) ;

et la couverture d'alimentation et de drainage (11) comprenant également un système d'alimentation et de drainage qui inclut une pompe externe, pour alimenter le récipient d'alimentation vertical et central (13) à travers un tube externe.

2. Le réacteur pour la formation de biofilms (3), selon la revendication 1, dans lequel le récipient vertical (12) est étanche et comprend :

- une forme tubulaire ; et
- un volume de travail incluant :
- un diamètre d'environ 27 mm à environ 108 mm ; et
- une longueur d'environ 136 mm à environ 544 mm,

et chaque récipient vertical (12) étant interconnecté à travers la base interconnectée (15) qui comprend un réservoir d'alimentation (23) ;

et chaque récipient vertical (12) est alimenté par le récipient d'alimentation vertical et central (13), qui est interconnecté à tous les récipients verticaux (12) à travers le réservoir d'alimentation (23) de la base interconnectée (15).

3. Le réacteur pour la formation de biofilms (3), selon l'une quelconque des revendications 1 ou 2, dans lequel les cylindres (9) comprennent :

- une forme cylindrique ;
- une surface d'échantillonnage ;
- un trou longitudinal (27), destiné à être inséré dans l'arbre vertical (8) ; et
- une section de fixation,

et les cylindres (9) étant fabriqués à l'aide d'un matériau choisi parmi la liste consistant en:

- -métaux ;

- alliages métalliques ;
- plastiques ;
- verre ; et
- revêtement et revêtements de surface, dans lequel les revêtements et revêtements de surface comprennent un nanomatériau ou une surface chimiquement modifiée ;

et les cylindres (9) sont fixés dans l'arbre vertical (8) ; et les cylindres (9) pivotent autour de leur propre axe.

4. Le réacteur pour la formation de biofilms (3), selon l'une quelconque des revendications 1 ou 2 ou 3, dans lequel la base interconnectée (15) comprend :

- une pluralité de cavités de base (28), pour fixer le récipient vertical respectif (12) ;
- une pluralité de cavités de base servant de support (29), pour fixer l'arbre de support vertical respectif (14) ;
- un trou de fuite horizontal (30), situé sur la face latérale de la base (15), pour vider le réservoir d'alimentation (23) ;
- une cavité centrale de base (31), pour fixer un récipient d'alimentation vertical et central (13) ; et
- le réservoir d'alimentation (23) situé immédiatement en-dessous de la cavité centrale de base (31) et également en-dessous de chacun des récipients verticaux respectifs (12).

5. Le réacteur pour la formation de biofilms (3) selon la revendication 4, dans lequel la base interconnectée (15) du réacteur pour la formation de biofilms (3) comprend les cavités de base (28) en nombre égal au nombre de récipients verticaux (12).

6. Le réacteur pour la formation de biofilms (3), selon l'une quelconque des revendications 1 ou 2 ou 3 ou 4 ou 5, dans lequel le système de pivot (10) comprend un moteur, pour pivoter une pluralité d'arbres verticaux (8), dans lequel le moteur pivote de manière synchrone chacun des arbres verticaux (8), et le système de pivot (10) comprend également :

- une poulie (32) fixée dans l'arbre vertical (8) ;
- un tendeur de poulie (33) ; et
- une ceinture de synchronisation qui interconnecte les quatre arbres verticaux (8) pour pivoter de manière synchrone chacun des arbres verticaux (8) à travers le moteur.

7. Un procédé de formation de biofilms, **caractérisé par** l'utilisation du réacteur pour la formation de biofilms (3) de l'une quelconque des revendications 1 ou 2 ou 3 ou 4 ou 5 ou 6 et comprenant les étapes suivantes :

i) nettoyer et désinfecter le réacteur pour la formation de biofilms à l'aide d'un agent chimique (3) ; et
ii) formation de biofilms à travers l'utilisation du réacteur pour la formation de biofilms (3), dans laquelle :

- l'étape de ii) formation de biofilms est réalisée sous condition opératoire par lots ; ou
- l'étape de ii) formation de biofilms est réalisée sous condition opératoire d'alimentation par lots ; ou
- l'étape de ii) formation de biofilms est réalisée sous condition opératoire continue.

8. Le procédé de formation de biofilms selon la revendication 7, dans lequel l'étape de ii) formation de biofilms comprend les sous-étapes suivantes :

ii.i) remplir les récipients verticaux (12), à travers le récipient d'alimentation vertical et central (13), avec la culture bactérienne (24) ;
ii.ii) remplir les récipients verticaux (12) à travers le récipient d'alimentation vertical et central (13), avec le milieu nutritif initial (25) ;
ii.iii) pour les conditions opératoires d'alimentation par lots ou continue, alimenter les récipients verticaux (12), à l'aide de la pompe externe (29) à travers un tube externe qui est directement lié au récipient d'alimentation vertical et central (13), avec un milieu nutritif dilué stérile (26) ; et
ii.iv) pivoter les cylindres (9) autour de leur propre axe, durant près de 24h à plusieurs mois et avec une rotation de 0 rpm à près de 2000 rpm.

Fig.1

(3)

(1)

(8)

(6)

(11)

(22)

(12)

(14)

(2)

(15)

(30)

Fig.2

(5)

(1)

(8)

(6)

(16)

(12')

(14')

(4)

(17)

Fig.3

(11)

(18) (20) (21)

(19)

(22)

Fig.4

(16)

(18') (20')

(21')

Fig.5

(15)

(28)

(29)

(31)

(30)

(23)

Fig.6

(17)

(28')

(29')

Fig.7

Fig. 8

(5)

(8)

(6)

(7)

(16)

(12')

(9)

(27)

(17)

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GOMES et al.** An overview on the reactors to study drinking water biofilms. *Water Research*, 2014, vol. 62, 63-87
- **GOMES et al.** Standardized reactors for the study of medical biofilms: a review of the principles and latest modifications. *Critical Reviews in Biotechnology*, 2018, vol. 38, 657-670
- **GOMES et al.** An overview on the reactors to study drinking water biofilms. *Water Research*, 2014, vol. 62, 63-87

- **JANG et al.** Measurement of chlorine dioxide penetration in dairy process pipe biofilms during disinfection. *Applied Microbiology and Biotechnology*, 2006, vol. 72, 368-376
- Use of flow cells and annular reactors to study biofilms. In: Biofilms in medicine, industry and environmental biotechnology: Characteristics, analysis and control. IWA Publishing, 2003
- **GOMES et al.** The action of chemical and mechanical stresses on single and dual species biofilm removal of drinking water bacteria. *Science of the Total Environment*, 2018, vol. 631-632, 987-993